# EUROPÄISCHE PATENTANMELDUNG

(11) **EP 1 382 352 A1**
(43) Veröffentlichungstag der Anmeldung: **21.01.2004**
(21) Anmeldenummer: 02016066.9
(22) Anmeldetag: 19.07.2002
(51) Int. Cl.: A61K 47/48

(54) **Bisacyloxypropylcystein-Konjugate und deren Verwendung**

(71) Anmelder: GBF Gesellschaft für Biotechnologische Forschung mbH, 38124 Braunschweig (DE)
(72) Erfinder: Mühlradt, Prof. Dr. Peter F., 38114 Braunschweig (DE); Morr, Dr. Michael, 38302 Wolfenbüttel (DE)
(74) Vertreter: Läufer, Martina, Dr.

(57) **Zusammenfassung**

Es werden neue Lipopeptid-Konjugate vorgeschlagen, bei denen ein doppelt fettsäuresubstituiertes Cystein über die Carboxylgruppe an einen gut wasserlöslichen und physiologisch verträglichen sowie nicht immunogenen polymeren Konjugatrest gebunden ist. Die neuen Konjugate zeigen sehr gute makrophagenstimulierende Wirkung und benötigen keine zusätzliche Lösungsvermittlung. Sie bieten sich für zahlreiche Verwendungen an, insbesondere zur Stimulierung von Makrophagen, zur Stimulierung der Antikörper-Synthese, zur Infektionsabwehr, zur Immunostimulanz, insbesondere gegenüber Tumoren, zur Vorbeugung und Behandlung von septischem Schock, zur Wundheilung und als Adjuvans für Impfstoffe.

## Beschreibung

Die Erfindung betrifft neue Bisacyloxypropylcystein-Konjugate und deren Verwendung, auch in Form pharmazeutischer Zusammensetzungen.

Es ist seit langem bekannt, dass bestimmte Lipopeptide Makrophagenaktivatoren sind (Hoffmann, P., S. Heinle, U.F. Schade, H. Loppnow, A.J. Ulmer, H.-D. Flad, G. Jung, and W. Bessler, 1988, "Stimulation of human and murine adherent cells by bacterial lipoprotein and synthetic Bisacyloxypropylcysteine analogues", Immunobiol. 177:158-170). Innerhalb dieser Klasse von Makrophagenaktivatoren sind insbesondere auch mehrfach an einem Propylcysteinrest fettsäuresubstituierte (acyloxysubstituierte) Peptide oder Proteine mit physiologischer Wirkung bekannt.

Peptide und darunter besonders Lipopeptide mit längeren Fettsäureketten haben jedoch für pharmazeutische und verwandte Anwendungen am menschlichen und tierischen Körper häufig den gravierenden Nachteil zu geringer Wasserlöslichkeit, was ihre Wirksamkeit und ihr Einsatzgebiet stark einschränkt.

Diese Probleme können durch Konjugieren der Proteine und Peptide an wasserlösliche Polymere gelöst werden. Aus der EP 0 510 356 B1 sind beispielsweise Polyethylenglykol-Proteinkonjugate bekannt, in denen ein Protein über einen Linker an Polyethylenglykol gebunden ist und hierdurch wesentlich wasserlöslicher gemacht wird. In der EP 0 510 356 B1 sind zahlreiche weitere Schriften genannt, in denen das Konjugieren von Peptiden oder Proteinen an wasserlösliche Polymere, hier besonders an Polyethylenglykol, beschrieben wird. Die Konjugation mit PEG wird heute auch als "Pegylieren" bezeichnet.

Auch sind bereits in oben beschriebener Weise pegylierte Makrophagenaktivatoren bekannt. Kommerziell erhältlich ist beispielsweise ein dreifach an einem Propyl-Cysteinrest acyloxy-substituiertes PEG, Tripalmitoyl-S-Glyceryl-Cystein-Polyethylenglykol, das als PAM₃-Cys-PEG bezeichnet werden kann.

PAM₃-Cys-PEG besitzt die Struktur 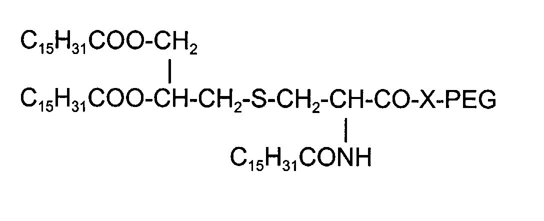 wobei das fettsäuresubstituierte Propylcystein an Position X über einen zweiwertigen Rest wie -NH-, -OCO-,
-S-, -O-, oder dergleichen mit dem Polyethylenglykol konjugiert ist.

Trotz der stark verbesserten Wasserlöslichkeit von PAM₃-Cys-PEG gegenüber den entsprechenden PAM₃-Cys-Peptiden ist bei der Verwendung dieser Substanz als Makrophagenaktivator die Zugabe eines organischen Lösungsvermittlers erforderlich oder sehr empfehlenswert, da die Makrophagenaktivierung sonst geringer wäre. Lösungsvermittler wie Octyl-Glucosid sind jedoch aus pharmakologische Sicht nicht ganz unproblematisch.

Es besteht daher weiterhin ein starkes Bedürfnis für neue, physiologisch gut verträgliche, insbesondere nicht-immunogene, gut wasserlösliche und aktive Makrophagenaktivatoren. Die Aufgabe der Erfindung besteht daher darin, einen solchen neuen gut verträglichen, wasserlöslichen und hochaktiven Makrophagenaktivator zur Verfügung zu stellen.

Die Aufgabe wird gelöst durch ein Bisacyloxypropylcystein-Konjugat gemäß Formel (1), 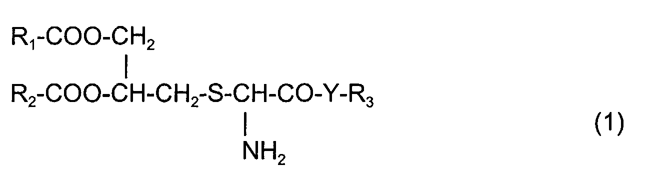 , wobei R₁ und R₂ gleich oder verschieden sein können und über die Carboxylgruppe gebundene Fettsäurereste bedeuten,
Y = -NH-, -O-, -S- oder -OCO- ist,
R₃ ein kovalent, ionisch oder assoziativ gebundener Konjugatrest ist, insbesondere ein wasserlösliches und physiologisch verträgliches, kovalent oder ionisch gebundenes Polymer, insbesondere kovalent gebundenes Polyethylenglykol (Polyoxyethylen),
-(CH₂-CH₂-O)ₘ-CH₂-CH₂-X,
mit X = OR, NR₂, SR, COOR und
R = H, Benzyl-, C₁₋₆-Alkyl, wobei mehrere Reste R gleich oder verschieden sein können,
ein Polyoxyethylen-Polyoxypropylen-Copolymer, ein Dextran, ein Zucker, ein Polyvinylpyrrolidon, ein Alginat, ein Pektin oder ein Kollagen,
und wobei der polymere Rest R₃ ein-, zwei- oder mehrfach mit 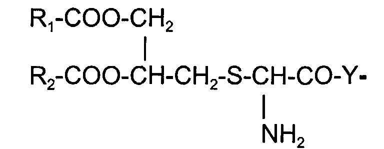 substituiert ist.

Bei dem erfindungsgemäßen Bisacyloxypropylcystein-Konjugat handelt es sich vorzugsweise um ein S-[2,3-bis(acyloxy)-(2S)-propyl]-L-cysteinyl-carboxypolyethylenglykol (BAP-Cys-PEG), bei dem das Polyethylenglykol über die Carboxylgruppe des Cysteins angebunden ist und die Aminogruppe des Cysteins frei bleibt. Unter Erhalt der Funktion bzw. hier der physiologischen Wirkung können dem Chemiker bekannte, übliche Modifikationen und Substitutionen an dem Molekül vorgenommen werden.

Die Reste R_{1,2} des erfindungsgemäßen Bisacyloxypropylcystein-Konjugats können gleich oder verschieden sein. Derzeit bevorzugt sind C₇₋₂₅-, vorzugsweise C₈₋₂₂-Alkyl, - Alkenyl oder -Alkinylgruppen, wobei die ungesättigten Positionen vorzugsweise in cis-Konfiguration vorliegen. Die Alkyl-, Alkenyl -und Alkinyl-Reste können verzweigte oder unverzweigte, cyclische oder cycloalkyl-substituierte Reste darstellen. Geeignete Reste R₁, R₂ sind aus der Fettsäurechemie hinlänglich bekannt.

Bezüglich der Gruppe Y kommt es lediglich darauf an, dass eine stabile Verbindung zu dem Konjugatrest R₃ hergestellt wird, damit das Bisacyloxypropylcystein hinlänglich an diesen gebunden und damit wasserlöslich bleibt.

Der Rest R₃ ist vorzugsweise ein Polyethylenglykol-Rest. Allgemein handelt es sich jedoch um einen kovalent, ionisch oder assoziativ gebundenen physiologisch verträglichen Konjugatrest, der geeignet ist, das Bisacyloxypropylcystein in eine aktive wasserlösliche Form zu überführen. Kovalent gebundene Polymere sind derzeit bevorzugt. Alternativ kann der Konjugatrest jedoch auch ein Dextran, ein Zucker, ein Polyvinylpyrrolidon, ein Alginat, ein Pektin oder ein Kollagen sein. Insbesondere Dextran ist als Blutexpander im Einsatz und von seiner physiologischen Verträglichkeit her unbedenklich.

Gerade im Falle hochpolymerer Konjugatreste können auch mehrere BAP-Cys-Einheiten an einen Konjugatrest R₃ gebunden sein.

Das Molekulargewicht des wasserlöslichen Polymerrestes ist vorzugsweise so gewählt ist, dass es pro Bisacyloxypropylcystein-Molekül 100 bis 30 000 Dalton beträgt.

Im Falle von Polyethylenglykol ist eine Kettenlänge m von 5 bis 700, vorzugsweise 100 bis 500 bevorzugt, jedoch nicht zwingend.

In bevorzugter Ausführungsform ist das S-[2,3-bis(acyloxy)-(2S)-propyl]-L-cysteinylcarboxy-polyethylenglykol ein S-[2,3-bis(palmitoyloxy)-(2S)-propyl]-L-cysteinylcarboxy-polyethylenglykol.

Das Bisacyloxypropylcystein-Konjugat nach dieser Erfindung weist gegenüber vorher bekannten Makrophagenaktivatoren den Vorteil auf, dass es eine gute Wasserlöslichkeit mit einer vergleichsweise sehr hohen Makrophagenaktivität verbindet (siehe unten). Es ist nicht immunogen, so dass bei der Verwendung an Mensch oder Tier keine Antikörperentwicklung gegen das Präparat erfolgt.

Die Erfindung umfasst weiterhin pharmazeutische Zusammensetzungen, die das erfindungsgemäße Bisacyloxypropylcystein-Konjugat enthalten. Derartige Zusammensetzungen schließen unter anderem Lösungen des erfindungsgemäßen Bisacyloxypropylcystein-Konjugats ein. Innerhalb dieser Zusammensetzungen können zusätzliche pharmazeutische Zusatz- und Hilfsstoffe enthalten sein. Insbesondere kann das Bisacyloxypropylcystein-Konjugat an einen pharmazeutisch akzeptablen Träger gebunden oder adsorbiert sein. Geeignete Träger sind dem Fachmann bekannt und stehen auch kommerziell zur Verfügung.

Die pharmazeutische Zusammensetzung liegt vorzugsweise in Form einer für die Injektion, für die Inhalation oder für die intranasale oder topische Applikation geeigneten Formulierung vor, wobei trägergebundene Applikationsformen eingeschlossen sind. Zur lokalen Applikation sind unter anderem Salben, Cremes, Tinkturen, Lösungen und der gleichen vorgesehen, wie dem Fachmann für diesen Zweck bekannt.

Die erfindungsgemäßen Bisacyloxypropylcystein-Konjugate oder die zugehörigen pharmazeutischen Zusammensetzungen können unter anderem zur Stimulierung von Makrophagen, zur Stimulierung der Antikörper-Synthese, zur Infektionsabwehr, zur Immunstimulanz, insbesondere gegenüber Tumoren, zur Vorbeugung und Behandlung von septischem Schock, zur Wundheilung und als Adjuvans für Impfstoffe eingesetzt werden.

Diese Verwendungen umfassen die Aktivierung von Makrophagen/Monozyten oder anderer Zellen, die die Rezeptorkombination Toll-like Rezeptor 2 und 6 tragen, mit allen indirekten Folgen durch Mediatoren in Tier oder Mensch. Dies impliziert die Anwendung als Adjuvans (also als Beimischung zu Vakzinen bzw. Impfstoffen), zur Tumortherapie im weitesten Sinne, einschließlich eines in vitro Primens zu reimplantierender autologer Zellen gegen Tumorantigene oder durch direkte Therapie, die lokal oder systemisch erfolgen kann, zur Erzeugung von Kreuztoleranz gegen Endotoxin und andere entsprechende mikrobielle Komponenten, was einen Schutz vor Sepsis ermöglicht, sowie zur Beschleunigung der Wundheilung.

### BEISPIELE

### Synthese eines erfindungsgemäßen Bisacyloxypropylcysteins mit R₁, R₂ = Palmitoyl, Y = NH and R₃ = PEG

Die Synthese von (I) erfolgt nach beschriebener Methode (Metzger, J., Wiesmüller, K.-H., Schaud, R., Bessler, W.G., und Jung, G., 1991, "Synthesis and novel immunologically active tripalmitoyl-S-glycerylcysteinyl Bisacyloxypropylcysteines as useful intermediates for immunogen preparations". Int. J. Pept. Protein Res. 37:46-57; Metzger,J.W., Wiesmüller, K.-H., und Jung, G., 1991, "Synthesis of N-Fmoc protected derivatives of S-(2,3-dihydroxypropyl)-cysteine and their application in peptide synthesis", Int. J. Pept. Protein Res. 38:545-554). 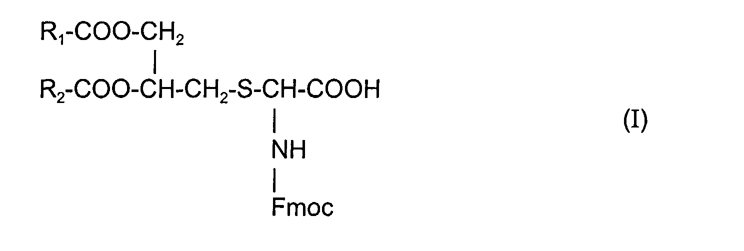 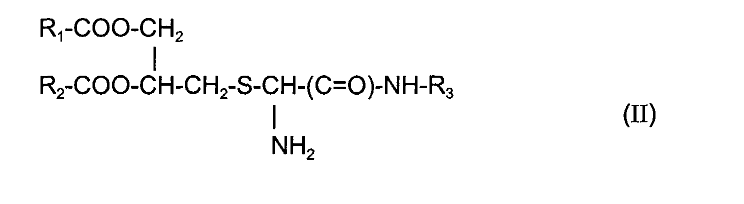 insbesondere mit: R_{1,2} = C₁₅H₃₁- (Palmitoyl-) und R₃ = -(CH₂-CH₂-O)ₓ-CH₂-CH₂-NH₂

Die Carboxylgruppe wird anschließend zum Beispiel mit wasserlöslichen NH₂-Gruppen enthaltenden Polymeren, z.B. Diamino-PEG nach bekannten Methoden (Carbodiimid-Synthese) verknüpft.

### Beispiel:

34 mg (38 µmol) (I) werden in 1 ml trockenem Dimethylformamid/Dichlormethan 2:1 gelöst und nacheinander mit 6 µl (38 µmol) Diisopropylcarbodiimid und 6 mg (38 µmol) 1-Hydroxybenzotriazol versetzt. Zu dieser Mischung gibt man 76 mg (38 µmol) Diamino-PEG 2000. Nach 24 Stunden bei Raumtemperatur wird zur Trocknung eingeengt und die Fmoc-Schutzgruppe mit 20 % Piperidin in Dimethylformamid abgespalten. Die Verbindung (II) wird durch Kieselgelchromatographie gereinigt. Charakterisierung durch NMR und Massenspektroskopie. Weitere Reinigung durch HPLC auf C18 Säule bei 40 °C, Puffer 1: 0,1 % TFA in Wasser; Puffer 2: 0,1 % TFA in 2-Propanol. Substanz eluiert bei ca. 80 % V/V Puffer 2.

Gehaltsanalyse erfolgt durch Fettsäurebestimmung mit intern zugemischtem Standard C14, nach Hydrolyse und GLC nach Standardmethoden oder durch Aminogruppenbestimmung mit Fluorescamin.

### Biologische Prüfung:

Prinzipiell kann die Makrophagen- und Monozytenaktivierung durch eine Vielzahl von Parametern gemessen werden, wie z.B. durch die Freisetzung von Zytokinen, Chemokinen oder Arachidonsäuremetaboliten in Kulturen von humanen Monozyten oder murinen Peritonealmakrophagen. Der hier verwendete Test beruht auf simultaner Stimulation von Peritonealmakrophagen der Maus mit Interferon-γ und Makrophagenaktivator, z.B. BAP-Cys-PEG zur Freisetzung von Stickstoffmonoxid. (Referenz: Mühlradt, P. F., and Frisch, M. ,1994, "Purification and partial biochemical characterization of a Mycoplasma fermentans-derived substance that activates macrophages to release nitric oxide, tumor necrosis factor, and interleukin-6", Infect. Immun. 62:3801-3807)

### Assay zur Stickstoffmonoxid-Freisetzung:

Kurzgefasst dienten Peritonealmakrophagen von C3H/HeJ-Mäusen als Makrophagenquelle. Diese wurden in 96-well Mikrotiterplatten kultiviert und simultan mit rIFN-γ und einer seriellen Verdünnung an Makrophagenaktivator stimuliert. Soweit erforderlich wurden die Makrophagenaktivatoren im ersten Verdünnungsschritt in 25 mM Octylglucosid gelöst und dann mit Medium weiterverdünnt. Nach einer Inkubationszeit von 45 - 48 Stunden, wurde das Nitrat mit Nitrat-Reduktase reduziert und die Ausgangssubstanz Stickstoffmonoxid mit Griess' Reagenz als Summe aus Nitrat und Nitrit bestimmt.

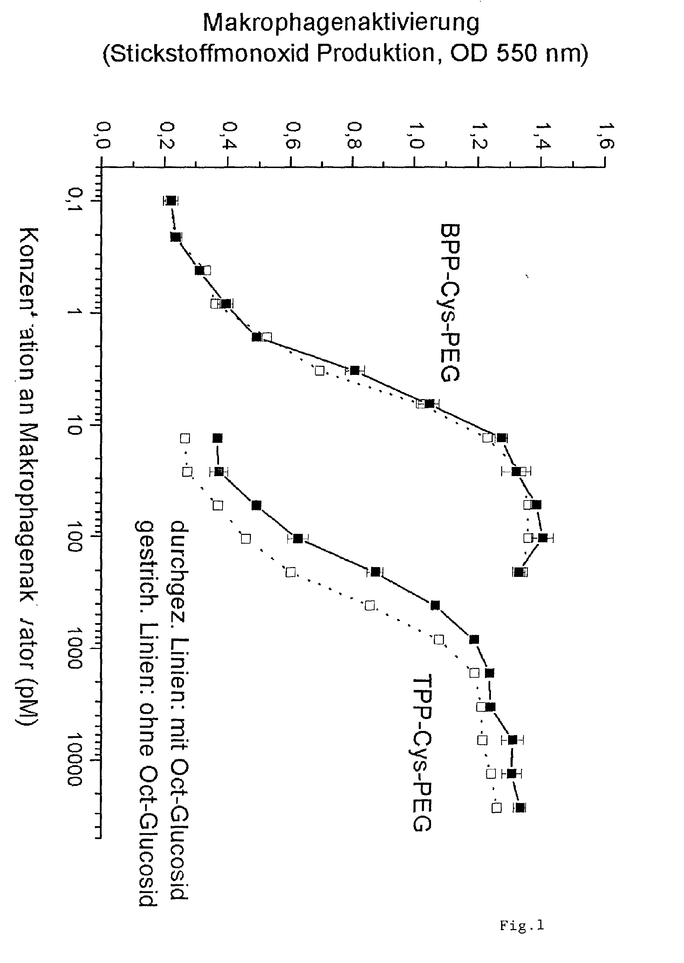

Die Ergebnisse des Makrophagenaktivierungstests sind in Figur 1 dargestellt.

Fig. 1: Konzentrationsabhängigkeit der Makrophagenaktivierung, gemessen mittels der Stickstoffmonoxid-Produktion (spektroskopisch bestimmt bei OD 550 nm), über der Konzentration an Makrophagenaktivator in Pikomol

Der Abbildung ist zu entnehmen, dass Bispalmitoyloxypropyl-Cystein-PEG (BPP-Cys-PEG) - ein Makrophagenaktivator gemäß dieser Erfindung - ein etwa zehnfach höheres Potential zur Aktivierung von Makrophagen besitzt als das bekannte PAM₃-Cys-PEG (hier als TPP-Cys-PEG bezeichnet ) Die Figur zeigt, dass das gleiche Ausmaß an Makrophagenaktivierung durch BPP-Cys-PEG schon bei einer zehnfach niedrigeren Konzentration als bei TPP-Cys-PEG erreicht wird.

Die Abbildung zeigt weiter, dass diese ausgezeichnete und unerwartete Aktivierungswirkung bei BPP-Cys-PEG durch Zugabe eines Lösungsvermittlers - hier Octyl-Glucosid - nicht merklich verbessert wird, während zur optimalen Entfaltung der Wirkung von PAM₃-Cys-PEG ein solcher Zusatz erforderlich ist.

Das neue Bisacyloxypropylcystein-Konjugat nach dieser Erfindung bedarf daher keiner zusätzlichen und gegebenenfalls physiologisch nachteiligen Lösungsvermittlung durch ein organisches Lösungsmittel oder Detergenz.

Ein weiterer Vorteil des BAP-Cys-PEG gegenüber dem PAM₃-Cys-PEG ist die größere Zellspezifität, die darauf zurückzuführen ist, dass diese Substanz die Kooperation der Toll-like-Rezeptoren 2 und 6 erfordert, während für eine Stimulation durch PAM₃-Cys-PEG die gleichzeitige Anwesenheit der Toll-like Rezeptoren 1 und 2 auf der zu stimulierenden Zelle ausreicht. Die Expression des Toll-like Rezeptor 6 ist auf bestimmte Zellen beschränkt, während Toll-like Rezeptor 1 ubiquitär von fast allen Körperzellen exprimiert wird.

## Patentansprüche

1. Bisacyloxypropylcystein-Konjugat gemäß Formel 1, 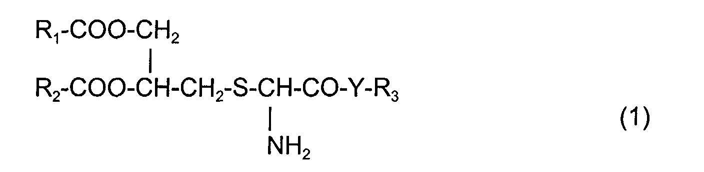 , wobei R₁ und R₂ gleich oder verschieden sein können und über die Carboxylgruppe gebundene Fettsäurereste bedeuten,
Y = -NH-, -O-, -S- oder -OCO- ist,
R₃ ein kovalent, ionisch oder assoziativ gebundener Konjugatrest ist, insbesondere ein wasserlösliches und physiologisch verträgliches, kovalent oder ionisch gebundenes Polymer, insbesondere kovalent gebundenes Polyethylenglykol (Polyoxyethylen),
-(CH₂-CH₂-O)ₘ-CH₂-CH₂-X,
mit X = OR, NR₂, SR, COOR und
R = H, Benzyl-, C₁₋₆-Alkyl, wobei mehrere Reste R gleich oder verschieden sein können,
ein Polyoxyethylen-Polyoxypropylen-Copolymer, ein Dextran, ein Zucker, ein Polyvinylpyrrolidon, ein Alginat, ein Pektin oder ein Kollagen,
und wobei der polymere Rest R₃ ein-, zwei- oder mehrfach mit 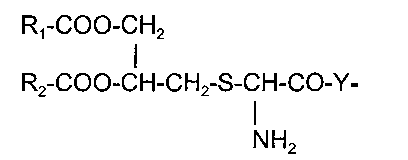 substituiert ist.

2. Bisacyloxypropylcystein-Konjugat nach Anspruch 1, **dadurch gekennzeichnet, dass** die Reste R_{1,2}, die gleich oder verschieden sein können, C₇₋₂₅-, vorzugsweise C₈₋₂₂-Alkyl, -Alkenyl oder -Alkinylgruppen sind und die ungesättigten Positionen vorzugsweise in cis-Konfiguration vorliegen, wobei die Alkyl-, Alkenyl -und Alkinyl-Reste verzweigte oder unverzweigte, cyclische oder cycloalkyl-substituierte Reste darstellen.

3. Bisacyloxypropylcystein-Konjugat nach Anspruch 1 oder 2, **dadurch gekennzeichnet, dass** das Molekulargewicht des wasserlöslichen Polymerrestes so gewählt ist, dass es pro Konjugat-Molekül 100 bis 30 000 Dalton beträgt.

4. Bisacyloxypropylcystein-Konjugat nach einem der Ansprüche 1 bis 3, **dadurch gekennzeichnet, dass** das Polyethylenglykol des Restes R₃ eine Kettenlänge m von 5 bis 700, vorzugsweise 100 bis 500 besitzt.

5. Bisacyloxypropylcystein-Konjugat nach einem der Ansprüche 1 bis 4, **dadurch gekennzeichnet, dass** die Verbindung ein S-[2,3-bis(acyloxy)-(2S)-propyl]-L-cysteinylcarboxy-polyethylenglykol ist, vorzugsweise S-[2,3-bis(palmitoyloxy)-(2S)-propyl]-Lcysteinyl-carboxy-polyethylenglykol.

6. Pharmazeutische Zusammensetzung, enthaltend ein Bisacyloxypropylcystein-Konjugat nach einem der Ansprüche 1 bis 5.

7. Pharmazeutische Zusammensetzung nach Anspruch 6, **dadurch gekennzeichnet, dass** sie pharmazeutische Zusatz- oder Hilfsstoffe und vorzugsweise einen pharmazeutisch verträglichen Träger enthält.

8. Pharmazeutische Zusammensetzung nach Anspruch 6 oder 7 in Form einer für die Injektion, für die Inhalation oder für die intranasale oder topische Applikation geeigneten Formulierung.

9. Verwendung der Bisacyloxypropylcystein-Konjugate nach einem der Ansprüche 1 bis 5 oder der pharmazeutischen Zusammensetzung nach Anspruch 6, 7 oder 8 zur Stimulierung von Makrophagen, zur Stimulierung der Antikörper-Synthese, zur Infektionsabwehr, zur Immunostimulanz, insbesondere gegenüber Tumoren, zur Vorbeugung und Behandlung von septischem Schock, zur Wundheilung und als Adjuvans für Impfstoffe.
